# EUROPÄISCHE PATENTSCHRIFT

(11) **EP 2 739 326 B1**
(45) Veröffentlichungstag und Bekanntmachung des Hinweises auf die Patenterteilung: **06.02.2019**
(21) Anmeldenummer: 12740902.7
(22) Anmeldetag: 30.07.2012
(51) Int. Cl.: A61M 1/36, A61M 5/14

(54) **Schlauchleitung mit einer Vorrichtung zum Fixieren eines Schlauchsegments der Schlauchleitung und eine Vorrichtung zur Überwachung der Zufuhr oder Abfuhr einer Flüssigkeit zu einem Patienten**
Tubing line with a device for fixing a tube segment of the tubing line and a device for monitoring the supply or removal of a fluid to or from a patient.
Conduite tubulaire avec un dispositif de fixation d'un segment de tuyau de la conduite tubulaire et un dispositif de surveillance de l'alimentation ou de l'évacuation d'un fluide vers un patient.

(30) Priorität: 04.08.2011 DE 102011109378; 04.08.2011 US 201161514912 P
(43) Veröffentlichungstag der Anmeldung: 11.06.2014
(73) Patentinhaber: Fresenius Medical Care Deutschland GmbH, 61352 Bad Homburg (DE)
(72) Erfinder: BONGERS, Alexander, 63225 Langen (DE)
(74) Vertreter: Oppermann, Frank
(86) Internationale Anmeldenummer: PCT/EP2012/003231
(87) Internationale Veröffentlichungsnummer: WO 2013/017252

(56) Entgegenhaltungen:
- EP-A1- 2 292 192
- DE-B3-102006 011 313
- FR-A- 567 345
- GB-A- 813 918
- US-A- 5 642 817

## Beschreibung

Die Erfindung betrifft eine Schlauchleitung zum Zuführen von einer Flüssigkeit zu einem Patienten oder Abführen einer Flüssigkeit von einem Patienten, insbesondere eine venöse Schlauchleitung eines extrakorporalen Blutkreislaufs, die eine venöse Punktionskanüle aufweist, oder eine arterielle Schlauchleitung eines extrakorporalen Blutkreislaufs, die eine arterielle Punktionskanüle aufweist, mit einer Vorrichtung zum Fixieren eines Schlauchsegments der Schlauchleitung in Form einer Schlaufe. Darüber hinaus betrifft die Erfindung eine Vorrichtung zur Überwachung der Zufuhr einer Flüssigkeit über eine Schlauchleitung zu einem Patienten und/oder der Abfuhr einer Flüssigkeit von einem Patienten, insbesondere zur Überwachung des Gefäßzugangs bei einer extrakorporalen Blutbehandlung, bei der Blut eines Patienten über eine venöse Schlauchleitung, die eine venöse Punktionskanüle aufweist, dem Patienten zugeführt und über eine arterielle Schlauchleitung, die eine arterielle Punktionskanüle aufweist, von dem Patienten abgeführt wird, wobei die Überwachungsvorrichtung eine Einrichtung zur Überwachung des Drucks in der Schlauchleitung aufweist, die derart ausgebildet ist, dass bei einer Veränderung des Drucks innerhalb vorgegebener Grenzen aufgrund einer Zugbeanspruchung der Schlauchleitung, insbesondere der arteriellen und/oder venösen Schlauchleitung, auf ein Herausrutschen der Kanüle der Schlauchleitung, insbesondere der arteriellen und/oder venösen Punktionskanüle der arteriellen bzw. venösen Schlauchleitung geschlossen wird.

Auf dem Gebiet der Medizintechnik ist eine Vielzahl von Einrichtungen bekannt, mit denen über eine Schlauchleitung einem Patienten Flüssigkeiten zugeführt oder Flüssigkeiten von dem Patienten abgeführt werden können. Dabei erfolgt der Zugang zu dem Patienten im Allgemeinen mit einem Katheter zum Einführen in Körperorgane oder einer Kanüle oder Nadel zum Punktieren von Gefäßen. Während der Untersuchung oder Behandlung ist ein ordnungsgemäßer Zugang zu dem Patienten sicherzustellen. Daher ist es erforderlich, den Patientenzugang zu überwachen.

Ein Anwendungsfall mit besonders hohen Anforderungen an die Sicherheit des Gefäßzuganges stellt die extrakorporale Blutbehandlung dar, bei der über eine arterielle Schlauchleitung, die eine arterielle Punktionskanüle aufweist, Blut von dem Patienten abgeführt, das Blut durch einen Dialysator geleitet wird und über eine venöse Blutleitung, die eine venöse Punktionskanüle aufweist, dem Patienten wieder zugeführt wird. Dabei besteht trotz regelmäßiger Überwachung des Patientenzugangs durch das Krankenhauspersonal grundsätzlich die Gefahr, dass die venöse Punktionskanüle unbemerkt aus dem Blutgefäß des Patienten herausrutscht. Während ein Herausrutschen der arteriellen Kanüle mit einem Ansaugen von Luft in die arterielle Schlauchleitung verbunden ist, das aufgrund einer maschinenseitigen Luftdetektion zu einem visuellen und/oder optischen Alarm und zur Unterbrechung der Behandlung führt, ist das Herausrutschen der venösen Kanüle und der damit gefürchtete Freifluss des Bluts in die Umgebung nicht ohne weiteres zu detektieren. Wenn das Herausrutschen der venösen Kanüle aber nicht sofort erkannt wird, kann der Patient verbluten.

Zur Lösung dieses Problems ist im Stand der Technik eine Vielzahl unterschiedlicher Vorrichtungen bekannt. Einige dieser Vorrichtungen greifen auf standardmäßig in den Blutbehandlungsmaschinen vorhandene Sicherheitsvorrichtungen zurück und lösen bei einem nicht ordnungsgemäßen Gefäßzugang eine sofortige Unterbrechung des extrakorporalen Blutkreislaufs aus. Die standardmäßig in den Behandlungsmaschinen vorhandenen Sicherheitsvorrichtungen basieren im Allgemeinen auf einer Überwachung des Drucks im extrakorporalen Blutkreislauf. In der Praxis hat sich jedoch gezeigt, dass allein mit einer Überwachung des Drucks im extrakorporalen Blutkreislauf das Herausrutschen insbesondere der venösen Punktionskanüle nicht mit der ausreichenden Sicherheit erkannt werden kann. Einige bekannte Sicherheitsvorrichtungen haben zwar eine ausreichende Sensitivität, sie reagieren aber sehr empfindlich auf Lageänderungen des Patienten, was oft zu Fehlalarmen führt. Nachteilig ist auch, dass sich die bestehenden Blutbehandlungsvorrichtungen nicht ohne weiteres mit den bekannten Überwachungsvorrichtungen nachrüsten lassen, sondern die Nachrüstung einen aufwendigen und kostenintensiven Eingriff in die Behandlungsmaschinen erfordert.

Aus der WO 2007/104350 A1 (DE 10 2006 011 313 B3) ist eine einfach zu handhabende, kostengünstig herstellbare und jederzeit nachrüstbare Vorrichtung bekannt, die eine sichere Überwachung eines Patientenzugangs erlaubt. Die Überwachung des Patientenzugangs beruht darauf, dass eine Schlaufe in der flüssigkeitsführenden Schlauchleitung gebildet wird. Es wird davon ausgegangen, dass ein Herausrutschen der Punktionskanüle oder des Katheters auf das Angreifen von Zugkräften an der Schlauchleitung zurückzuführen ist. Wenn die Schlauchleitung auf Zug beansprucht wird, zieht sich die Schlaufe zwangsläufig zu. Dies führt zu einem erhöhten Druckverlust in der Schlauchleitung, der leicht zu detektieren ist.

Zum Fixieren der Schlauchleitung in Form einer Schlaufe finden besondere Fixierungsmittel Verwendung. In der WO 2007/104350 A1 werden zwei alternative Ausführungsformen beschrieben, die sich dadurch unterscheiden, dass die Fixierungsmittel bei der einen Ausführungsform der Schlauchleitung lose beigelegte Fixierungsstücke sind, während bei der anderen Ausführungsform die Fixierungsstücke einstückiger Bestandteil des Katheters für den Patientenzugang sind. Die lose beigelegten Fixierungsstücke zeichnen sich durch zwei Ösen aus, die der Aufnahme jeweils eines Abschnitts der Schlauchleitung dienen, wobei die Schlauchleitung in Form einer Schlaufe fixiert wird.

Mit den bekannten Fixierungsstücken kann ein Schlauchsegment der Schlauchleitung in Form einer Schlaufe fixiert werden, die sich bei einer Zugbeanspruchung zusammenzieht. Nachteilig ist jedoch, dass die hierzu erforderliche Zugkraft relativ hoch ist.

Die EP 2 292 192 A1 beschreibt ein Fixierungs-System zum Fixieren eines Blasenkatheters in Form einer Schlaufe, das ein oder zwei ringförmige Körper aufweist, um den Katheter mit einer flexiblen Länge ordnungsgemäß lagern zu können. Zur Einstellung der erforderlichen Länge des Katheters soll mit dem Fixierungs-System eine Schlaufe gebildet werden. Dabei soll der Katheter aber stets offen bleiben, so dass die Flüssigkeitsströmung nicht unterbrochen ist. Daher muss sichergestellt sein, dass sich die Schlaufe nicht zusammenziehen kann, wodurch die Flüssigkeitsströmung unterbrochen wäre.

Aus der FR 567 345 A und der GB 813 918 A sind schließlich Gürtelschnallen bekannt, die über zwei ringförmige Körper verfügen, wobei das eine Ende des Gürtels fest mit den ringförmigen Körpern verbunden ist und das andere Ende des Gürtels zwischen den ringförmigen Körpern klemmend gehalten wird.

Der Erfindung liegt die Aufgabe zugrunde, eine Vorrichtung und ein Verfahren zum Fixieren eines Schlauchsegments einer Schlauchleitung in Form einer Schlaufe zu schaffen, mit der sich die für das Zusammenziehen der Schlaufe erforderlichen Zugkräfte reduzieren lassen.

Die Lösung dieser Aufgabe erfolgt mit den Merkmalen der unabhängigen Patentansprüche. Die abhängigen Ansprüche betreffen bevorzugte Ausführungsformen der Erfindung.

Die erfindungsgemäße Vorrichtung beruht auf der Verwendung von der Kombination eines Führungsstücks und eines Sicherungsstücks. Mit dem Führungsstück werden die sich kreuzenden Abschnitte des ein Auge bildenden Schlauchsegments derart fixiert, dass das Auge eine Schlaufe bildet, die sich bei einer Zugbeanspruchung zusammenzieht. Mit dem Sicherungsstück wird der zwischen den sich kreuzenden Abschnitten liegende Abschnitt des das Auge bildenden Schlauchsegments derart fixiert, dass beim Zuziehen der Schlaufe der zwischen den sich kreuzenden Abschnitten liegende Abschnitt des das Auge bildenden Schlauchsegments nicht durch das Führungsstück rutscht.

Das Führungsstück und das Sicherungsstück können ein gemeinsames Teil bilden oder als getrennte Teile ausgebildet sein. Das Führungsstück ist als ein ringförmiger Körper ausgebildet, der die sich kreuzenden Abschnitte des ein Auge bildenden Schlauchsegments umschließt. Das Führungsstück muss nicht als ein kreisringförmiger Körper ausgebildet sein. Grundsätzlich ist jede von der Kreisform abweichende Form, bspw. eine Ellipse oder ein Rechteck möglich. Vorzugsweise ist aber der ringförmige Körper ein kreisringförmiger Körper, in dem die sich kreuzenden Abschnitte des ein Auge bildenden Schlauchsegments lose geführt sind.

Wenn die Schlauchleitung auf Zug beansprucht wird, zieht sich die Schlaufe zusammen, wobei die sich kreuzenden Abschnitte des Schlauchsegments in dem ringförmigen Körper des Führungsstücks lose geführt sind. Entscheidend ist, dass sich beim Zuziehen der Schlaufe der ringförmige Körper um die sich kreuzenden Abschnitte des Schlauchsegments herumdreht. Dadurch wird die Reibung des Schlauchsegments in dem ringförmigen Körper stark reduziert, sodass deutlich geringere Zugkräfte erforderlich sind, um die Schlaufe zuzuziehen.

Die Schlaufe zieht sich solange zusammen, bis das Sicherungsstück den Abschnitt des Sicherungsstücks des Schlauchsegments zurückhält, der zwischen den sich kreuzenden Abschnitten der Schlaufe liegt. Dadurch wird das Schlauchsegment plötzlich abgeknickt. Dies führt zu einem deutlich erhöhten Druckverlust in der Schlauchleitung, der leicht zu detektieren ist.

Bei einer bevorzugten Ausführungsform ist der ringförmige Körper des Führungsstücks derart ausgebildet, dass das Führungsstück zum Einführen der Schlauchleitung aufklappbar und nach dem Einführen der Schlauchleitung wieder verschließbar ist. Diese Ausführungsform erleichtert das Anlegen des Führungsstücks an der Schlauchleitung, um das Auge des Schlauchsegments zu einer Schlaufe zu fixieren. Vorzugsweise besteht der ringförmige Körper des Führungsstücks aus zwei Teilstücken, wobei das eine Ende des ersten Teilstücks und das eine Ende des zweiten Teilstücks beweglich miteinander verbunden sind, und das andere Ende des ersten Teilstücks und das andere Ende des zweiten Teilstücks einrastend oder einschnappend miteinander verbindbar sind. Eine besonders bevorzugte Ausführungsform sieht für die bewegliche Verbindung der Enden der beiden Teilstücke ein Filmscharnier vor. Die einrastende oder einschnappende bzw. bewegliche Verbindung der beiden Teilstücke sollte so beschaffen sein, dass das Führungsstück einen ringförmigen Körper bildet, der im geschlossenen Zustand eine glatte Oberfläche hat, an der die sich kreuzenden Abschnitte des Schlauchsegments beim Zusammenziehen der Schlaufe entlang gleiten können.

Anstelle eines aufklappbaren Führungsstücks ist auch ein Führungsstück in Form eines geschlossenen ringförmigen Körpers möglich. Bei dieser Ausführungsform kann allerdings das Führungsstück nicht um die sich kreuzenden Abschnitte des Schlauchsegments gelegt werden. Zur Bildung der Schlaufe wird bei dieser Ausführungsform ein umgeknicktes Schlauchsegment der Schlauchleitung durch den ringförmigen Körper des Führungsstücks geführt.

Für das Sicherungsstück zur Sicherung des Schlauchsegments in dem Führungsstück sieht die Erfindung zwei alternative Ausführungsformen vor.

Bei der einen Ausführungsform ist das Sicherungsstück als ein ringförmiger Körper ausgebildet, der den Abschnitt des ein Auge bildenden Schlauchsegments umschließt. Zum Einführen der Schlauchleitung kann auch das Sicherungsstück aufklappbar und nach dem Einführen der Schlauchleitung wieder verschließbar ausgebildet sein. Grundsätzlich ist es aber auch möglich, ein als geschlossener ringförmiger Körper ausgebildetes Sicherungsstück schon vor dem Anlegen des Führungsstücks auf die Schlauchleitung aufzuziehen.

Bei der alternativen Ausführungsform ist das Sicherungsstück als ein langgestreckter Körper ausgebildet, wobei die Enden des langgestreckten Körpers des Sicherungsstücks an dem ringförmigen Körper des Führungsstücks derart befestigt sind, dass die Enden des langgestreckten Körpers entlang des ringförmigen Körpers des Sicherungsstücks verschiebbar sind.

Bei der ersten alternativen Ausführungsform ist der ringförmige Körper des Sicherungsstücks, der die Schlaufe locker umschließt, nicht mit dem ringförmigen Körper des Führungsstücks verbunden, während bei der zweiten alternativen Ausführungsform der langgestreckte Körper des Sicherungsstücks mit dem ringförmigen Körper des Führungsstücks verbunden ist. Bei beiden Ausführungsformen hindert das Sicherungsstück die Schlaufe daran, vollständig durch das Führungsstück hindurch gezogen zu werden. Bei der ersten Ausführungsform wird der ringförmige Körper des Sicherungsstücks von der sich zusammenziehenden Schlaufe einfach mitgenommen, während bei der zweiten alternativen Ausführungsform sich der ringförmige Körper des Sicherungsstücks frei in dem Führungsstück oder umgekehrt drehen kann, so dass die Schlaufe nicht am Zusammenziehen gehindert wird.

Das als langgestreckter Körper ausgebildete Sicherungsstück wird vorzugsweise erst dann mit dem ringförmigen Körpers des Führungsstücks verbunden, wenn das Schlauchsegment die Schlaufe bildet. Eine bevorzugte Ausführungsform sieht daher vor, dass wenigstens eine der beiden Enden des langgestreckten Körpers des Sicherungsstücks lösbar an dem ringförmigen Körper des Führungsstücks befestigt ist. Vorzugsweise sind beide Enden des langgestreckten Körpers des Sicherungsstücks lösbar an dem ringförmigen Körper des Führungsstücks befestigt, so dass das Führungsstück vollständig von dem Sicherungsstück abgenommen werden kann und das Sicherungsstück beim Bilden der Schlaufe nicht im Wege steht. Grundsätzlich ist es aber auch möglich, dass sich das Sicherungsstück nicht von dem Führungsstück lösen lässt. In diesem Fall ist es aber erforderlich, eine oder beide Enden der Schlauchleitung auf beiden Seiten des Sicherungsstücks durch das Führungsstück zu ziehen, um die Schlaufe zu bilden.

Mit der erfindungsgemäßen Vorrichtung kann eine Vorrichtung zum Zu- oder Abführen von Flüssigkeiten zu einem oder von einem Patienten jederzeit nachgerüstet werden.

Die bekannten Blutbehandlungsvorrichtungen verfügen bereits über eine Einrichtung zur Überwachung des Drucks im extrakorporalen Blutkreislauf. Wenn der Druck vorgegebene Grenzen überschreitet, kann die Blutbehandlungsvorrichtung einen Alarm geben und/oder die Blutbehandlung unterbrechen. Die hierzu erforderlichen mechanischen Einrichtungen sind in den bekannten Blutbehandlungsvorrichtungen vorhanden. Daher ist es lediglich erforderlich, die vorgegebenen Grenzwerte für den Druck im extrakorporalen Blutkreislauf auf die verwendete Schlauchleitung abzustimmen.

Von Vorteil ist, dass nicht nur ein fehlerhafter Patientenzugang leicht erkannt werden kann, sondern dass die Bildung einer Schlaufe gleichsam die Übertragung von Zugkräften auf den Katheter oder die Kanüle schwächen kann. Wenn der Schlauch auf Zug beansprucht wird, zieht sich zunächst die Schlaufe zu, so dass die Zugkräfte nicht sofort auf den Katheter oder die Kanüle übertragen werden. Erst wenn sich die Schlaufe so weit zugezogen hat, dass der Schlauch abknickt, werden die Zugkräfte auf den Katheter oder die Kanüle übertragen. Dann setzt aber schon der Schutzmechanismus ein. Es reicht bereits ein leichter Knick aus, um eine Erhöhung des Druckverlusts in der Schlauchleitung sicher detektieren zu können. Außerdem ist vorteilhaft, dass sich der Staudruck mit zunehmender Strömungsgeschwindigkeit der Flüssigkeit schneller aufbaut. Dies ist insbesondere dann der Fall, wenn die Schlauchleitung aus einem flexiblen Material besteht, das leicht nachgibt.

Wenn die Schlauchlänge entsprechend großzügig bemessen und dem Patienten ein gewisser Bewegungsfreiraum gegeben wird, entstehen nur wenige Fehlalarme, weil im Rahmen üblicher Bewegungen keine oder nur geringe Zugkräfte am Blutschlauch auftreten können. Zudem ist es möglich, die Vorrichtung zum Zu- oder Abführen der Flüssigkeit bei einem Störfall nicht sofort abzuschalten, sondern nur einen Alarm auszulösen. Sollte Alarm ausgelöst werden, kann das Krankenhauspersonal den Knick durch Zurückführen der Schlauchleitung in die Schlaufenform beseitigen, ohne dass die Behandlung unterbrochen werden muss.

Grundsätzlich wäre es ohne weitere Hilfsmittel möglich, die Blutschlauchleitung einfach als Auge zu legen und eines der beiden freien Schlauchenden durch das Auge hindurchzuführen, so dass sich die Schlaufe unter Zugbelastung zuziehen kann. Es hat sich herausgestellt, dass es bei den am Markt üblichen Blutschlauchleitungen bereits beim Durchführen eines freien Schlauchendes durch eine solches Auge zum unbeabsichtigten Knicken der Blutschlauchleitung kommen kann, wodurch die Blutschlauchleitung für die Dialysebehandlung so nicht mehr verwendbar wäre. Außerdem hat sich gezeigt, dass zum Zuziehen einer so erzeugten Schlaufe unter Zugbelastung aufgrund der bei den üblichen Blutschlauchwerkstoffen hohen Reibung zwischen den unmittelbar aufeinanderliegenden Blutschlauchleitungsabschnitten erheblich höhere Zugkräfte erforderlich wären, als dies bei der vorliegenden Erfindung der Fall ist. Damit besteht die Gefahr, dass unter Zugbelastung die Kanüle aus des Fistel bzw. dem Shunt des Patienten herausrutscht, bevor sich die Schlaufe zuziehen kann. Erfindungsgemäß sollen aber bereits geringe Zugkräfte ein Zuziehen der Schlaufe bewirken. Die genannten Nachteile werden durch die vorliegende Erfindung überwunden, ohne dass dazu neue Blutschlauchleitungen entwickelt werden müssen, die mit den genannten Nachteile, insbesondere vorzeitiges Knicken und hohe Reibung, verbunden sind.

Im Folgenden werden verschiedene Ausführungsbeispiele der Erfindung unter Bezugnahme auf die Zeichnungen näher erläutert.

Es zeigen:
Fig. 1 die wesentlichen Komponenten einer Blutbehandlungsvorrichtung zusammen mit der erfindungsgemäßen Vorrichtung zur Überwachung des Patientenzugangs in stark vereinfachter schematischer Darstellung,
Fig. 2 ein erstes Ausführungsbeispiel der erfindungsgemäßen Vorrichtung zum Fixieren eines Schlauchsegments einer der Schlauchleitungen der Blutbehandlungsvorrichtung von Fig. 1, wobei die Schlauchleitung vor dem Zusammenziehen der Schlaufe dargestellt ist,
Fig. 3 die Vorrichtung zum Fixieren eines Schlauchsegments von Fig. 2, wobei die Schlaufe der Schlauchleitung zugezogen ist,
Fig. 4 das Führungsstück oder das Sicherungsstück der Vorrichtung zum Fixieren der Schlauchleitung von Fig. 2 in der Draufsicht,
Fig. 5 das Führungsstück oder Sicherungsstück von Fig. 4 in geschnittener Darstellung,
Fig. 6 eine alternative Ausführungsform der erfindungsgemäßen Vorrichtung zum Fixieren eines Schlauchsegments einer der Schlauchleitungen der
Blutbehandlungsvorrichtung von Fig. 1, wobei die Schlauchleitung vor dem Zusammenziehen der Schlaufe dargestellt ist,
Fig. 7 die Vorrichtung zum Fixieren eines Schlauchsegments von Fig. 6, wobei die Schlaufe der Schlauchleitung zugezogen ist, und
Fig. 8 ein Ausführungsbeispiel der alternativen Ausführungsform der Vorrichtung zum Fixieren eines Schlauchsegments.

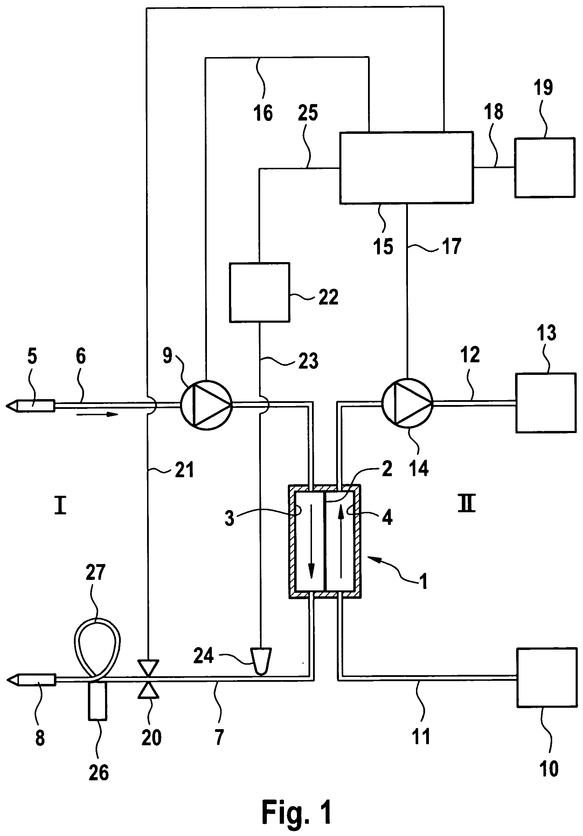

Fig. 1 zeigt die wesentlichen Komponenten einer Hämodialysevorrichtung, die über eine Vorrichtung zur Überwachung des venösen Gefäßzugangs verfügt. Die Hämodialysevorrichtung weist einen Dialysator 1 auf, der durch eine semipermeable Membran 2 in eine Blutkammer 3 und eine Dialysierflüssigkeitskammer 4 unterteilt ist. An der Fistel oder dem Shunt des Patienten ist mittels einer arteriellen Punktionskanüle 5 eine arterielle Schlauchleitung 6 angeschlossen, die zu dem Einlass der Blutkammer 3 des Dialysators führt. Von dem Auslass der Blutkammer 3 des Dialysators 1 geht eine venöse Schlauchleitung 7 ab, die mittels einer venösen Punktionskanüle 8 an der Fistel oder dem Shunt des Patienten angeschlossen ist. Die arterielle Schlauchleitung 6 ist in eine okkludierende Blutpumpe 9 eingelegt, die das Blut im extrakorporalen Blutkreislauf I fördert.

Der Dialysierflüssigkeitskreislauf II der Hämodialysevorrichtung umfasst eine Dialysierflüssigkeitsquelle 10, an der eine Dialysierflüssigkeitszuführleitung 11 angeschlossen ist, die zu dem Einlass der Dialysierflüssigkeitskammer 4 des Dialysators führt. Von dem Auslass der Dialysierflüssigkeitskammer 4 des Dialysators 1 geht eine Dialysierflüssigkeitsabführleitung 12 ab, die zu einem Auslass 13 führt. In die Dialysierflüssigkeitsabführleitung 12 ist eine Dialysierflüssigkeitspumpe 14 geschaltet. Die Steuerung der Dialysevorrichtung übernimmt eine zentrale Steuereinheit 15, die über Steuerleitungen 16, 17 die Blut- und Dialysierflüssigkeitspumpe 9, 14 ansteuert. Die zentrale Steuereinheit 15 ist über eine Datenleitung 18 mit einer Alarmeinheit 19 verbunden, die bei einem Störfall einen optischen und/oder akustischen Alarm gibt.

Stromab der Blutkammer 3 des Dialysators 1 befindet sich an der venösen Schlauchleitung 7 eine elektromagnetisch betätigbare Schlauchklemme 20, die über eine weitere Steuerleitung 21 von der zentralen Steuereinheit 15 geschlossen wird, wenn die venöse Punktionskanüle (Nadel) durch eine Zugkraft am Schlauch aus dem Gefäßzugang des Patienten heraus rutschen sollte. Darüber hinaus stoppt die Steuereinheit 15 nach dem Herausrutschen der Kanüle die Blutpumpe 9.

Zur Überwachung des Drucks in der venösen Schlauchleitung 7 weist die Dialysevorrichtung eine Überwachungseinrichtung 22 auf, die über eine Datenleitung 23 mit einem Drucksensor 24 verbunden ist, der den Druck in der venösen Schlauchleitung 7 misst. Die Drucküberwachungseinrichtung 22 kommuniziert mit der zentralen Steuereinheit 15 über eine weitere Datenleitung 25.

Die Vorrichtung zur Überwachung des venösen Gefäßzugangs weist die nachfolgend noch im Einzelnen beschriebene Vorrichtung 26 zum Fixieren eines Schlauchsegments der venösen Schlauchleitung 7 in Form einer Schlaufe 27 auf. Die Schlaufe wird stromauf der venösen Punktionskanüle 8 vorzugsweise an einem Abschnitt der Schlauchleitung gebildet, in dem die Schlauchleitung noch am Körper des Patienten, beispielsweise an dessen Unterarm, anliegt.

Während der Dialysebehandlung wird die venöse Schlauchleitung 7 nicht auf Zug beansprucht. Die Drucküberwachungseinrichtung 22 misst einen Druck P in der venösen Schlauchleitung, der innerhalb vorgegebener Grenzen liegt. Die typischen venösen Drücke liegen bei etwa 100 bis 200 mmHg. Es sei angenommen, dass die Druckmesseinrichtung 22 einen venösen Druck von 150 mmHg misst. Bei derartigen Druckverhältnissen wird ein Grenzwertfenster mit einer Breite von 100 mmHg definiert, wobei der untere Grenzwert für den Druck bei 100 mmHg und der obere Grenzwert bei 200 mmHg liegt. Liegt der gemessene Druck ober- bzw. unterhalb der vorgegebenen Grenzen von 100 bzw. 200 mmHg, löst die zentrale Steuereinheit 15 einen optischen und/oder akustischen Alarm aus.

Wenn an der venösen Schlauchleitung 7 stromauf der Schlaufe 27 gezogen wird, besteht die Gefahr, dass die Punktionskanüle 8 aus der Vene des Patienten herausrutscht. Bleibt dies unbemerkt, besteht für den Patienten Lebensgefahr. Der Druckabfall in der venösen Schlauchleitung durch Verlust des Innendrucks im Patientenzugang von etwa 15 bis 25 mmHg kann aber nicht zu einer Unterschreitung des unteren Grenzwertes für den venösen Druck von 100 mmHg führen, so dass ohne die erfindungsgemäße Überwachungsvorrichtung das Herausrutschen der venösen Punktionskanüle nicht erkannt wird.

Da die Schlauchleitung aber oberhalb der Punktionskanüle eine Schlaufe bildet, führt das Herausrutschen der Punktionskanüle durch eine Zugkraft am Schlauch zu der Auslösung eines Alarms und/oder der Unterbrechung der Blutbehandlung. Wenn an der venösen Schlauchleitung 7 gezogen wird, zieht sich die Schlaufe 27 zunächst zusammen, so dass sich die Zugspannung zunächst "abfedern" lässt. Eine weitere Zugbeanspruchung führt jedoch dazu, dass sich die Schlaufe 27 so lange zusammenzieht, bis der Schlauch letztlich abknickt. Da sich das Blut an der Knickstelle staut, steigt der venöse Druck in der Schlauchleitung stromauf der Knickstelle. Der Staudruck hängt von dem verbleibenden offenen Querschnitt des abgeknickten Schlauchsegments an der Knickstelle und vom Blutfluss ab. Die Druckmesseinrichtung 22 misst mit dem stromauf der Schlaufe 27 angeordneten Drucksensor 24 den Druck vor der Knickstelle. Innerhalb kurzer Zeit, ca. 1 bis 2 Sekunden, steigt der Druck von 150 mmHg aufgrund der Verengung an der Knickstelle stark an, wodurch der obere Grenzwert von 200 mmHg für den venösen Druck überschritten wird. Folglich löst die zentrale Steuereinheit 15 einen Alarm aus und unterbricht die Blutbehandlung durch Schließen der venösen Schlauchklemme 20 und Stoppen der Blutpumpe 9.

Für die Überwachung können in der Steuerung verschiedene Grenzwerte unterschiedlicher Höhe vorgegeben werden. Wenn der gemessene Druck beispielsweise einen ersten vorgegebenen Grenzwert überschreitet, kann darauf geschlossen werden, dass die Schlaufe teilweise zusammengezogen ist und die Punktionskanüle wahrscheinlich herausrutscht, und es kann ein Voralarm ausgelöst werden. Wenn der gemessene Druck beispielsweise einen zweiten Grenzwert, der größer ist als ein erster Grenzwert, überschreitet, kann darauf geschlossen werden, dass der Schlauch abgeknickt ist und die Punktionskanüle aus dem Patientenzugang herauszurutschen droht.

Da eine nennenswerte Druckänderung beim Zuziehen der Schlaufe erst relativ spät, d.h. erst mit der Knickbildung auftritt, muss sichergestellt werden, dass die Schlauchleitung tatsächlich abknickt. Da aufgrund der Knickbildung der Schlauchleitung, die bei üblichen Schlauchleitungen erst bei weit fortgeschrittenem Zuziehen der Schlaufe auftritt, eine gesicherte Grenzüberschreitung des gemessenen Drucks erfolgt, kann es vorteilhaft sein, wenn eine frühe Knickbildung begünstigt wird. Die Sensitivität der Überwachungseinrichtung kann so erhöht werden. Es ist vorteilhaft, dies durch eine konstruktive oder strukturelle Anisotropie der Schlauchleitung zu unterstützen, wobei die Form und/oder Beschaffenheit des Materials des Schlauches an der Knickstelle abweichend von der Form und/oder Beschaffenheit des Materials außerhalb der Knickstelle verändert wird. So kann beispielsweise eine Sollknickstelle dadurch geschaffen werden, dass die Schlauchwandung dünner ausgebildet wird oder beispielsweise von der runden Querschnittsform abweicht. So kann die runde Schlauchleitung an der Knickstelle beispielsweise einen elliptischen Querschnitt aufweisen.

Nachfolgend wird ein erstes Ausführungsbeispiel der Vorrichtung 26 zum Fixieren eines Schlauchsegments der Schlauchleitung in Form einer Schlaufe beschrieben, die an eine bestehende Schlauchleitung einer konventionellen Dialysemaschine jederzeit angebracht werden kann oder bereits an der Schlauchleitung der Dialysemaschine vorhanden sein kann. Da die bekannten Dialysemaschinen im Allgemeinen bereits über eine Einrichtung zur Überwachung des Drucks verfügen, die beim Über- und/oder Unterschreiten von vorgegebenen Grenzwerten reagieren, brauchen weitere Vorrichtungen nicht vorgesehen zu werden, um den Patientenzugang überwachen zu können.

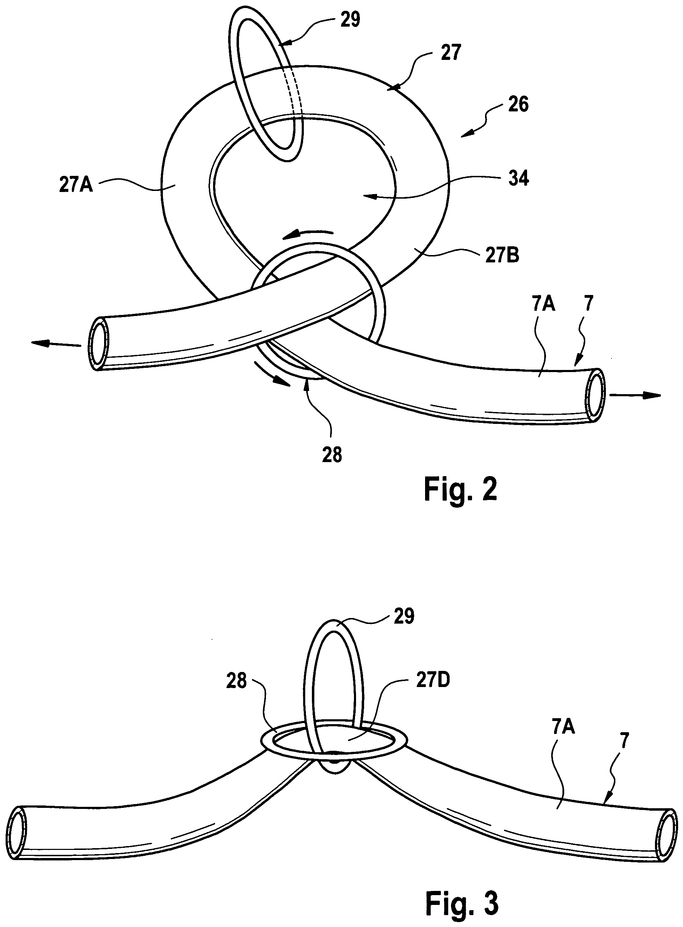

Die Vorrichtung 26 zum Fixieren eines Schlauchsegments der Schlauchleitung 7 in Form einer Schlaufe 27 (Fig. 1) besteht aus einem Führungsstück 28 und einem Sicherungsstück 29 (Fig. 2). Bei dem ersten Ausführungsbeispiel ist sowohl das Führungsstück 28 als auch das Sicherungsstück 29 als ein ringförmiger Körper ausgebildet. Das Führungsstück 28 und Sicherungsstück 29 sind bei dem vorliegenden Ausführungsbeispiel kreisringförmige Körper, die nachfolgend bei diesem Ausführungsbeispiel als Führungsring 28 und Sicherungsring 29 bezeichnet werden.

Fig. 2 zeigt den Führungsring 28 und den Sicherungsring 29 zusammen mit dem Segment 7A der Schlauchleitung 7, das in Form einer Schlaufe 27 fixiert ist. Der Führungsring 28 fixiert die sich kreuzenden Abschnitte 27A, 27B des ein Auge 34 bildenden Schlauchsegments 7A der Schlauchleitung 7 derart, dass sich die Schlaufe 27 bildet. Wenn an einem oder beiden Enden der Schlauchleitung 7 gezogen wird, zieht sich die Schlaufe 27 zusammen. Der Sicherungsring 29 verhindert, dass sich die bei einer Zugbeanspruchung zusammenziehende Schlaufe 27 durch den Führungsring 28 rutscht. Darüber hinaus bewirkt der Sicherungsring 29, dass der Schlauch unter Zugbeanspruchung abknickt, so dass aufgrund der Knickstelle 27D eine leicht zu detektierende Druckänderung erfolgt. Das abgeknickte Schlauchsegment 7A der Schlauchleitung 7 ist zusammen mit dem Führungs- und Sicherungsring 28, 29 in Fig. 3 dargestellt.

Beim Zuziehen der Schlaufe 27 werden die sich kreuzenden Abschnitte 27A, 27B des Schlauchsegments 7A der Schlauchleitung 7 von dem Führungsring 28 geführt und fixiert. Während sich die Schlaufe 27 zusammenzieht, dreht sich der Führungsring 28 um die Schlauchkreuzung 27A, 27B herum. Dadurch wird die Reibung des Schlauchsegments in dem Führungsring deutlich reduziert. Die Schlaufe 27 zieht sich so lange zu, bis der Sicherungsring 29 auf dem Führungsring 28 aufliegt. Dabei schließen der Sicherungs- und Führungsring einen Winkel von etwa 90° ein. Die entstehende Knickstelle 27D liegt im Zentrum des Führungsrings 28 oberhalb des Sicherungsrings 29. Da der Sicherungsring 29 das Schlauchsegment beim Zusammenziehen der Schlaufe locker umschließt, hindert der Sicherungsring 29 das Schlauchsegment nicht daran, sich zusammen zu ziehen. Die für das Zusammenziehen der Schlaufe erforderliche Zugkraft ist daher gering.

Für eine besonders reibungsarme Funktion sollte der Innendurchmesser des Führungsrings 28 etwas größer sein als das Doppelte des Außendurchmessers der Schlauchleitung 7. Die erfindungsgemäße Vorrichtung wird vorzugsweise an den Abschnitten der in der Dialyse verwendeten Schlauchleitungen angebracht, die sich an den Kanülen befinden. Diese Schlauchabschnitte haben typischerweise einen Außendurchmesser von 5,5 mm. Bei einer derartigen Schlauchleitung hat sich ein Führungsring mit einem Innendurchmesser von etwa 14 mm als vorteilhaft erwiesen. Der Innendurchmesser des Führungsrings sollte zwischen 10 und 20 mm liegen. Dabei hat sich eine Dicke d des Führungsrings von 1 - 2 mm, insbesondere 1,5 mm als vorteilhaft erwiesen.

Der Außendurchmesser des Sicherungsrings 29 sollte so bemessen sein, dass der Sicherungsring 29 beim Zusammenziehen der Schlaufe 27 nicht durch den Führungsring 28 hindurchgezogen werden kann. Es hat sich als vorteilhaft erwiesen, wenn der Außendurchmesser des Sicherungsrings 29 etwas größer als der Außendurchmesser des Führungsrings 28 ist. Auch hat sich als vorteilhaft erwiesen, wenn die Dicke des Sicherungsrings 29 etwas größer als die Dicke des Führungsrings 28 ist. Die Oberflächen des Führungsrings 28 und des Sicherungsrings 29 sollten möglichst glatt beschaffen sein, so dass die Schlauchleitung in den Ringen 28, 29 gleiten kann.

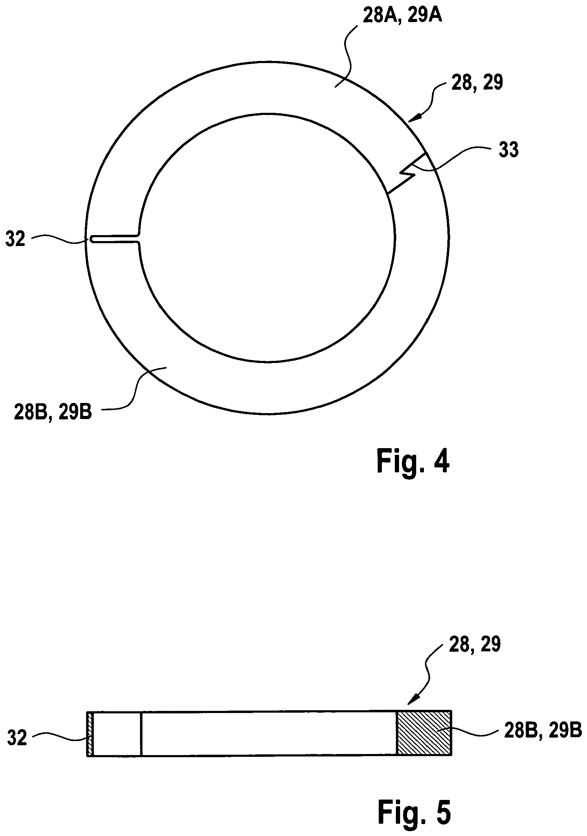

Die Fig. 4 und 5 zeigen eine besonders bevorzugte Ausfiihrungsform, in der sowohl der Führungsring 28 als auch der Sicherungsring 29 ausgebildet sein kann. Der Vorteil dieser Ausführungsform liegt darin, dass der Führungs- bzw. Sicherungsring 28, 29 nicht auf die Schlauchleitung aufgezogen zu werden braucht, oder die Schlauchleitung durch den Ring gezogen werden muss, sondern sich der Ring um die Schlauchleitung legen lässt. Der Führungs- bzw. Sicherungsring 28, 29 besteht aus zwei Teilstücken 28A bzw. 29A und 28B bzw. 29B. Das eine Ende des ersten Teilstücks 28A, 29A und das eine Ende des zweiten Teilstücks 28B, 29B sind mit einem Scharnier 32, insbesondere einem Filmscharnier, miteinander verbunden, das in den Fig. 4 und 5 nur andeutungsweise dargestellt ist. Die anderen Enden der beiden Teilstücke sind mit einer ebenfalls nur andeutungsweise dargestellten Rast- oder Schnappverbindung 33 miteinander verbunden. Nach Lösen der Rast- oder Schnappverbindung 33 kann der Führungs- oder Sicherungsring 28, 29 somit aufgeklappt und um die Schlauchleitung 7 gelegt werden.

Wenn sowohl der Führungs- als auch der Sicherungsring 28, 29 aufklappbar sind, braucht die Schlauchleitung nur zu einem Auge geformt und der Sicherungsring 29 angelegt zu werden.

Wenn der Führungsring 28 ein geschlossener Ring und der Sicherungsring 29 ein aufklappbarer Ring ist, wird die Schlauchleitung 7 zunächst abgeknickt. Das abgeknickte Teilstück der Schlauchleitung wird dann durch den geschlossenen Sicherungsring geführt. Anschließend wird der aufgeklappte Sicherungsring 29 um die Schlauchleitung gelegt und geschlossen. Wenn beide Ringe 28, 29 geschlossene Ringe sind, muss die Schlauchleitung durch beide Ringe gezogen werden, so dass sie eine Schlaufe bildet (Fig. 2).

Vorzugsweise sollten die Ringe 28, 29 nach dem Legen der Kanülen zu Beginn der Dialysebehandlung an der Schlauchleitung angebracht und erst zum Ende der Dialysebehandlung wieder abgenommen werden. Die Ringe sollten an dem Teil der Schlauchleitung angebracht werden, an dem die Dialysekanüle angeschlossen ist, da der mit der Dialyse verbundene Schlauch dünner und somit flexibler als der des angeschlossenen Blutschlauchsystems ist.

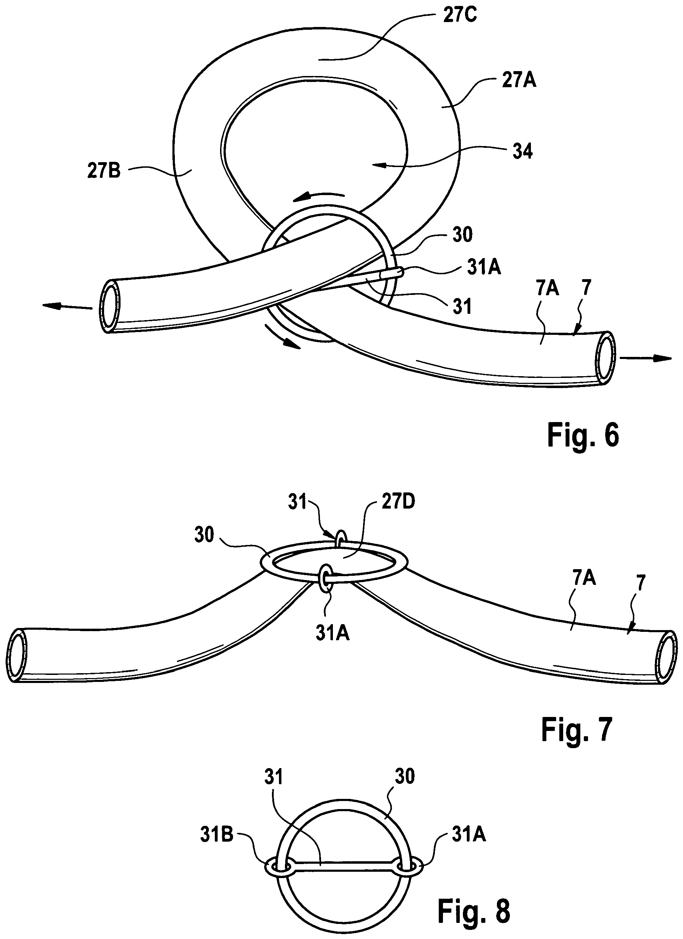

Die Figuren 6 und 7 zeigen eine alternative Ausfiihrungsform der Vorrichtung zum Fixieren eines Schlauchsegments der Schlauchleitung in Form einer Schlaufe vor und nach dem Zusammenziehen der Schlaufe. Auch die alternative Ausfiihrungsform verfügt über ein Führungsstück 30, das bei dem vorliegenden Ausführungsbeispiel wieder ein Führungsring 30 ist. Die alternative Ausführungsform unterscheidet sich nur durch die Ausbildung des Sicherungsstücks 31. Bei der alternativen Ausführungsform ist das Sicherungsstück 31 als langgestreckter Körper ausgebildet, der an dem Führungsring 30 festgelegt ist.

Bei dem vorliegenden Ausführungsbeispiel ist das Sicherungsstück ein Steg 31, dessen beide Enden 31A, 31B verschiebbar an dem Führungsring 30 befestigt sind (Fig. 8). Die beiden Enden 31A, 31B des Sicherungsstegs 31 können bspw. als Laschen ausgebildet sein, die den Führungsring 30 lose umschließen. Dabei teilt der Sicherungssteg 31 den Führungsring 30 in zwei Hälften auf, durch die sich die kreuzenden Abschnitte 27A, 27B des Schlauchsegments 27 erstrecken.

Von Vorteil ist, wenn eine oder beide Enden 31A, 31B des Sicherungsstegs 31 lösbar mit dem Führungsring 30 verbunden sind. Als lösbare Verbindungen stehen sämtliche dem Fachmann bekannte Verbindungen zur Verfügung. Wenn beide Enden 31A, 31B des Sicherungsstegs 31 lösbar mit dem Führungsring 30 verbunden sind, kann der Sicherungssteg 31 vollständig abgenommen werden. Wenn nur eine der beiden Enden des Sicherungsstegs 31 mit dem Führungsring 30 lösbar verbunden ist, kann der Sicherungssteg seitlich umgeklappt werden. In beiden Fällen wird das Anlegen des Sicherungsrings vereinfacht.

Die Länge des Sicherungsstegs 31 entspricht dem Durchmesser des Führungsrings 30. Aufgrund der losen Führung des Sicherungsstegs 31 kann sich der Führungsring 30 beim Zusammenziehen der Schlaufe 27 drehen, während der Führungssteg seine Position zwischen den sich kreuzenden Abschnitten 27A, 27B des Schlauchsegments 27 beibehält oder umgekehrt. Die Schlaufe 27 zieht sich unter Zugbeanspruchung solange zu, bis das Schlauchsegment 27C, das zwischen den sich kreuzenden Abschnitten 27A, 27B liegt, auf den Sicherungssteg 31 gezogen wird, wodurch die Schlauchleitung an der Knickstelle 27D abknickt (Fig. 7).

Die Vorrichtung lässt sich leicht anlegen, indem zunächst der Sicherungssteg 31 abgenommen oder umgeklappt und das abgeknickte Teilstück der Schlauchleitung 27 durch den Führungsring 30 gezogen wird. Anschließend wird der Sicherungssteg an dem Führungsring montiert. Hierzu ist es entweder erforderlich beide oder nur eines der beiden Enden 31A, 31B des Führungsstegs 31 mit dem Sicherungsring zu verbinden.

Der Führungssteg 31 kann grundsätzlich unterschiedlich ausgebildet sein. Von Vorteil ist, wenn der Führungssteg 31 in der Kreisebene des Führungsrings 30 schmal ausgebildet ist, um die Halbkreisflächen, in denen die Schlauchschleife geführt wird, nicht übermäßig zu verkleinern. In Längsrichtung der Schlauchleitung sollte der Steg breiter ausgebildet sein, um ein Knicken des Schlauchs sicherzustellen. Auf jeden Fall sollte der Führungssteg derart profiliert sein, dass eine Beschädigung der Schlauchleitung beim Abknicken verhindert wird.

Führungs- und Sicherungsstück 28, 29 bzw. 30, 31 können als ein zur einmaligen Verwendung bestimmtes Produkt ausgebildet sein. In diesem Fall bieten sich als Werkstoffe höherfeste Kunststoffe, z. B. PC oder POM an. Um eine Wiederverwendung des zur einmaligen Verwendung bestimmten Produkts auszuschließen, können die Rast- und Schnappverbindung 33 des Sicherungs- oder Führungsstücks derart beschaffen sein, dass sich die beiden Teilstücke nur einmal miteinander verbinden lassen. Geeignete Verbindungsmechanismen sind dem Fachmann bekannt. Wenn das Führungs- und Sicherungsstück wieder verwendbar sein soll, können Führungs- oder Sicherungsstück aus Edelstahl bestehen. Insbesondere für das Führungsstück bietet sich aufgrund der hohen Festigkeit Edelstahl an.

## Patentansprüche

1. Schlauchleitung zum Zuführen von einer Flüssigkeit zu einem Patienten oder Abführen einer Flüssigkeit von einem Patienten, insbesondere eine venöse Schlauchleitung (7) eines extrakorporalen Blutkreislaufs, die eine venöse Punktionskanüle aufweist, oder eine arterielle Schlauchleitung eines extrakorporalen Blutkreislaufs, die eine arterielle Punktionskanüle aufweist, mit einer Vorrichtung zum Fixieren eines Schlauchsegments der Schlauchleitung in Form einer Schlaufe
**dadurch gekennzeichnet, dass**
die Vorrichtung (26) zum Fixieren eines Schlauchsegments in Kombination aufweist:
ein als ringförmiger Körper ausgebildetes Führungsstück (28, 30) zur Fixierung der sich kreuzenden Abschnitte des ein Auge (34) bildenden Schlauchsegments derart, dass das Auge eine Schlaufe (27) bildet, die sich bei einer Zugbeanspruchung zusammenzieht, und
ein Sicherungsstück (29, 31) zur Fixierung des zwischen den sich kreuzenden Abschnitten (27A, 27B) liegenden Abschnitts des das Auge (34) bildenden Schlauchsegments derart, dass beim Zuziehen der Schlaufe (27) der zwischen den sich kreuzenden Abschnitten liegende Abschnitt des das Auge bildenden Schlauchsegments nicht durch das ringförmige Führungsstück (28, 30) rutscht,
wobei die sich kreuzenden Abschnitte (27A, 27B) des ein Auge (34) bildenden Schlauchsegments der Schlauchleitung in das als ringförmiger Körper ausgebildete Führungsstück (28) eingelegt sind, so dass das Auge eine Schlaufe bildet, die sich bei einer Zugbeanspruchung zusammenzieht, und das Sicherungsstück (29) den zwischen den sich kreuzenden Abschnitten liegenden Abschnitt des das Auge bildenden Schlauchsegments fixiert, so dass beim Zuziehen der Schlaufe der zwischen den sich kreuzenden Abschnitten liegende Abschnitt des das Auge bildenden Schlauchsegments nicht durch das ringförmige Führungsstück rutscht.

2. Schlauchleitung nach Anspruch 1, **dadurch gekennzeichnet, dass** der ringförmige Körper des Führungsstücks (28, 30) derart ausgebildet ist, das das Führungsstück (28, 30) zum Einführen der Schlauchleitung aufklappbar und nach dem Einführen der Schlauchleitung wieder verschließbar ist.

3. Schlauchleitung nach Anspruch 1 oder 2, **dadurch gekennzeichnet, dass** der ringförmige Körper des Führungsstücks (28) aus zwei Teilstücken (28A, 28B) besteht, wobei das eine Ende des ersten Teilstücks (28)A und das eine Ende des zweiten Teilstücks (28B) beweglich miteinander verbunden sind, und das andere Ende des ersten Teilstücks (28A) und das andere Ende des zweiten Teilstücks (28B) einrastend oder einschnappend miteinander verbindbar sind.

4. Schlauchleitung nach Anspruch 3, **dadurch gekennzeichnet, dass** das eine Ende des ersten Teilstücks (28A) und das eine Ende des zweiten Teilstücks (28B) des Führungsstücks (28) mit einem Scharnier (32), insbesondere mit einem Filmscharnier, beweglich miteinander verbunden sind.

5. Schlauchleitung nach einem der Ansprüche 1 bis 4, **dadurch gekennzeichnet, dass** das Sicherungsstück (29) als ein ringförmiger Körper ausgebildet ist.

6. Schlauchleitung nach Anspruch 5, **dadurch gekennzeichnet, dass** der ringförmige Körper des Sicherungsstücks (29) derart ausgebildet ist, dass das Sicherungsstück (29) zum Einführen der Schlauchleitung aufklappbar und nach dem Einführen der Schlauchleitung wieder verschließbar ist.

7. Schlauchleitung nach Anspruch 6, **dadurch gekennzeichnet, dass** der ringförmige Körper des Sicherungsstücks (29) aus zwei Teilstücken (29A, 29B) besteht, wobei das eine Ende des ersten Teilstücks (29A) und das eine Ende des zweiten Teilstücks (29B) beweglich miteinander verbunden sind, und das andere Ende des ersten Teilstücks (29A) und das andere Ende des zweiten Teilstücks (29B) einrastend oder einschnappend miteinander verbindbar sind.

8. Schlauchleitung nach Anspruch 7, **dadurch gekennzeichnet, dass** das eine Ende des ersten Teilstücks (29A) und das eine Ende des zweiten Teilstücks des Sicherungsstücks (29B) mit einem Scharnier (32), insbesondere mit einem Filmscharnier, beweglich miteinander verbunden sind.

9. Schlauchleitung nach einem der Ansprüche 1 bis 4, **dadurch gekennzeichnet, dass** das Sicherungsstück (31) als ein langgestreckter Körper ausgebildet ist, wobei die Enden des langgestreckten Körpers des Sicherungsstücks (31) an dem ringförmigen Körper des Führungsstücks (30) derart befestigt sind, dass die Enden des langgestreckten Körpers entlang des ringförmigen Körper des Führungsstücks verschiebbar sind.

10. Schlauchleitung nach Anspruch 9, **dadurch gekennzeichnet, dass** ein oder beide Enden (31A, 31B) des langgestreckten Körpers des Sicherungsstücks (31) lösbar an dem ringförmigen Körper des Führungsstücks (30) beweglich befestigt sind.

11. Vorrichtung zur Überwachung der Zufuhr einer Flüssigkeit über eine Schlauchleitung zu einem Patienten und/oder der Abfuhr einer Flüssigkeit von einem Patienten, insbesondere zur Überwachung des Gefäßzugangs bei einer extrakorporalen Blutbehandlung, bei der Blut eines Patienten über eine venöse Schlauchleitung, die eine venöse Punktionskanüle aufweist, dem Patienten zugeführt und über eine arterielle Schlauchleitung, die eine arterielle Punktionskanüle aufweist, von dem Patienten abgeführt wird, wobei die Überwachungsvorrichtung (22) eine Einrichtung zur Überwachung des Drucks in der Schlauchleitung aufweist, die derart ausgebildet ist, dass bei einer Veränderung des Drucks innerhalb vorgegebener Grenzen aufgrund einer Zugbeanspruchung der Schlauchleitung, insbesondere der arteriellen und/oder venösen Schlauchleitung, auf ein Herausrutschen der Kanüle der Schlauchleitung, insbesondere der arteriellen und/oder venösen Punktionskanüle der arteriellen bzw. venösen Schlauchleitung geschlossen wird,
**dadurch gekennzeichnet, dass** die Überwachungsvorrichtung eine Schlauchleitung nach einem der Ansprüche 1 bis 10 aufweist.

## Claims

1. Tubing for feeding a fluid to a patient or feeding a fluid out of a patient, and in particular a venous tubing (7) of an extra-corporeal blood circuit which venous tubing has a venous puncturing canula, or an arterial tubing of an extra-corporeal blood circuit which arterial tubing has an arterial puncturing canula, comprising a device for fixing a segment of the tubing in the form of a loop,
**characterised in that** the device (26) for fixing a segment of the tubing, in combination, comprising:
a guiding piece (28, 30) taking the form of an annular body for fixing the intersecting portions of the segment of tubing which forms an eye (34) in such a way that the eye forms a loop (27) which contracts under tractive stress, and
a securing piece (29, 31) for fixing that portion of the segment of tubing forming the eye (34) which is situated between the intersecting portions (27A, 27B) in such a way that, when the loop (27) tightens, that portion of the segment of tubing forming the eye which is situated between the intersecting portions does not slip through the annular guiding piece (28, 30),
the intersecting portions (27A, 27B) of that segment of the tubing which forms an eye (34) being placed in the guiding piece (28) taking the form of an annular body, the eye thus forming a loop which contracts under a tractive stress, and the securing piece (29) fixing that portion of the segment of tubing forming the eye which is situated between the intersecting portions, so that that portion of the segment of tubing forming the eye which is situated between the intersecting portions does not slip through the annular guiding piece when the loop tightens.

2. Device according to claim 1, **characterised in that** the annular body forming the guiding piece (28, 30) is so designed that the guiding piece (28, 30) can be folded open to allow the tubing to be inserted and can be closed again once the tubing has been inserted.

3. Device according to claim 1 or 2, **characterised in that** the annular body forming the guiding piece (28) comprises two part-pieces (28A, 28B), with one end of the first part-piece (28A) and one end of the second part-piece (28B) being connected together to be movable and the other end of the first part-piece (28A) and the other end of the second part-piece (28B) being able to be connected together by latching or snapping in.

4. Device according to claim 3, **characterised in that** one end of the first part-piece (28A) of the guiding piece (28) and one end of the second part-piece (28B) thereof are connected together to be movable by a hinge (32) and in particular by a film hinge.

5. Device according to one of claims 1 to 4, **characterised in that** the securing piece (29) takes the form of an annular body.

6. Device according to claim 5, **characterised in that** the annular body forming the securing piece (29) is so designed that the guiding piece (28) can be folded open to allow the tubing to be inserted and can be closed again once the tubing has been inserted.

7. Device according to claim 6, **characterised in that** the annular body forming the securing piece (29) comprises two part-pieces (29A, 29B), with one end of the first part-piece (29A) and one end of the second part-piece (29B) being connected together to be movable and the other end of the first part-piece (29A) and the other end of the second part-piece (29B) being able to be connected together by latching or snapping in.

8. Device according to claim 7, **characterised in that** one end of the first part-piece (29A) of the securing piece and one end of the second part-piece (29B) thereof are connected together to be movable by a hinge (32) and in particular by a film hinge.

9. Device according to one of claims 1 to 4, **characterised in that** the securing piece (31) takes the form of an elongated body, with the ends of the elongated body forming the securing piece (31) being fastened to the annular body forming the guiding piece (30) in such a way that the ends of the elongated body can be displaced along the annular body forming the guiding piece.

10. Device according to claim 9, **characterised in that** one or both ends (31A, 31B) of the elongated body forming the securing piece (31) are releasably fastened to the annular body forming the guiding piece (30) to be movable.

11. Arrangement for monitoring the infeed of a fluid via tubing to a patient and/or the outfeed of a fluid from a patient, and in particular for monitoring the vascular access in extra-corporeal blood treatment in which a patient's blood is fed to the patient via venous tubing which has a venous puncturing canula and is fed out from the patient via arterial tubing which has an arterial puncturing canula, the monitoring arrangement (22) comprising a means for monitoring the pressure in the tubing which is so designed that if there is change in pressure within preset limits due to a tractive stress on the tubing, and in particular on the arterial and/or venous tubing, is it concluded that the canula on the tubing, and in particular the arterial and/or venous puncturing canula on the arterial or venous tubing respectively is slipping out, **characterised in that** the monitoring arrangement comprises a segment of tubing according to one of claims 1 to 10.

## Revendications

1. Conduite tubulaire pour l'acheminement d'un fluide à un patient ou l'évacuation d'un fluide d'un patient, en particulier une conduite tubulaire veineuse (7) d'une circulation sanguine extracorporelle qui présente une canule de ponction veineuse, ou une conduite tubulaire artérielle d'une circulation sanguine extracorporelle qui présente une canule de ponction artérielle, avec un dispositif de fixation d'un segment tubulaire de la conduite tubulaire sous la forme d'une boucle
**caractérisée en ce que**
le dispositif (26) de fixation d'un segment tubulaire présente en combinaison :
une pièce de guidage (28, 30) constituée en tant que corps annulaire pour la fixation de tronçons se croisant d'un segment tubulaire formant un oeil (34) de telle sorte que l'oeil forme une boucle (27) qui se resserre sous l'effet d'une contrainte de traction, et
une pièce de sécurité (29, 31) pour la fixation du tronçon se trouvant entre les tronçons (27A, 27B) se croisant du segment tubulaire formant l'oeil (34) de telle sorte que lors de la traction de la boucle (27), le tronçon se trouvant entre les tronçons se croisant du segment tubulaire formant l'oeil ne glisse pas au travers de la pièce de guidage annulaire (28, 30), dans laquelle les tronçons se croisant (27A, 27B) du segment tubulaire formant un oeil (34) de la conduite tubulaire sont insérés dans la pièce de guidage (28) constituée en tant que corps annulaire, de sorte que l'oeil forme une boucle qui se resserre sous l'effet d'une contrainte de traction, et que la pièce de sécurité (29) fixe le tronçon se trouvant entre les tronçons se croisant du segment tubulaire formant l'oeil de sorte que lors de la traction de la boucle, le tronçon se trouvant entre les tronçons se croisant du segment tubulaire formant l'oeil ne glisse pas au travers de la pièce de guidage annulaire.

2. Conduite tubulaire selon la revendication 1, **caractérisée en ce que** le corps annulaire de la pièce de guidage (28, 30) est constitué de telle sorte que la pièce de guidage (28, 30) peut être ouverte pour l'introduction de la conduite tubulaire et puisse être refermée après l'introduction de la conduite tubulaire.

3. Conduite tubulaire selon la revendication 1 ou 2, **caractérisée en ce que** le corps annulaire de la conduite de guidage (28) est constitué de deux portions (28A, 28B), dans laquelle l'une extrémité de la première portion (28A) et l'une extrémité de la seconde portion (28B) sont reliées l'une à l'autre de façon mobile, et l'autre extrémité de la première portion (28A) et l'autre extrémité de la seconde portion (28B) peuvent être reliées l'une à l'autre par emboîtement ou encliquetage.

4. Conduite tubulaire selon la revendication 3, **caractérisée en ce que** l'une extrémité de la première portion (28A) et l'une extrémité de la seconde portion (28B) de la pièce de guidage (28) sont reliées l'une à l'autre de façon mobile avec une charnière (32), en particulier avec une charnière à film.

5. Conduite tubulaire selon l'une quelconque des revendications 1 à 4, **caractérisée en ce que** la pièce de sécurité (29) est constituée en tant que corps annulaire.

6. Conduite tubulaire selon la revendication 5, **caractérisée en ce que** le corps annulaire de la pièce de sécurité (29) est constitué de telle sorte que la pièce de sécurité (29) peut être ouverte pour l'introduction de la conduite tubulaire et puisse être refermée après l'introduction de la conduite tubulaire.

7. Conduite tubulaire selon la revendication 6, **caractérisée en ce que** le corps annulaire de la pièce de sécurité (29) est constitué de deux portions (29A, 29B), dans laquelle l'une extrémité de la première portion (29A) et l'une extrémité de la seconde portion (29B) sont reliées l'une à l'autre de façon mobile, et l'autre extrémité de la première portion (29A) et l'autre extrémité de la seconde portion (29B) peuvent être reliées l'une à l'autre par emboîtement ou encliquetage

8. Conduite tubulaire selon la revendication 7, **caractérisée en ce que** l'une extrémité de la première portion (29A) et l'une extrémité de la seconde portion de la pièce de sécurité (29B) sont reliées l'une à l'autre de façon mobile avec une charnière (32), en particulier avec une charnière à film.

9. Conduite tubulaire selon l'une quelconque des revendications 1 à 4, **caractérisée en ce que** la pièce de sécurité (31) est constituée en tant que corps étendu longitudinalement, dans laquelle les extrémités du corps étendu longitudinalement de la pièce de sécurité (31) sont fixées au corps annulaire de la pièce de guidage (30) de telle sorte que les extrémités du corps étendu longitudinalement peuvent être déplacées le long du corps annulaire de la pièce de guidage.

10. Conduite tubulaire selon la revendication 9, **caractérisée en ce qu'**une ou les deux extrémités (31A, 31B) du corps étendu longitudinalement de la pièce de sécurité (31) sont fixées de façon mobile détachable au corps annulaire de la pièce de guidage (30).

11. Dispositif de surveillance de l'acheminement d'un fluide par une conduite tubulaire à un patient et/ou de l'évacuation d'un fluide d'un patient, en particulier pour la surveillance de l'accès vasculaire lors d'un traitement extracorporel du sang, lors duquel du sang d'un patient est acheminé au patient par une conduite tubulaire veineuse qui présente une canule de ponction veineuse et est évacué du patient par une conduite tubulaire artérielle qui présente une canule de ponction artérielle, dans lequel le dispositif de surveillance (22) présente un système de surveillance de la pression dans la conduite tubulaire qui est constitué de telle sorte que lors d'une modification de la pression dans des limites prédéterminées en raison d'une contrainte de traction de la conduite tubulaire, en particulier de la conduite tubulaire artérielle et/ou veineuse, un glissement de la canule de la conduite tubulaire, en particulier de la canule de ponction artérielle et/ou veineuse de la conduite tubulaire artérielle ou veineuse est déduit,
**caractérisé en ce que** le dispositif de surveillance présente une conduite tubulaire selon l'une quelconque des revendications 1 à 10.
